# EUROPÄISCHE PATENTSCHRIFT

(11) **EP 3 230 731 B1**
(45) Veröffentlichungstag und Bekanntmachung des Hinweises auf die Patenterteilung: **07.08.2019**
(21) Anmeldenummer: 15834790.6
(22) Anmeldetag: 08.12.2015
(51) Int. Cl.: G01N 31/12, G01N 33/18

(54) **ANALYSEANORDNUNG ZUR WASSER- UND ABWASSERANALYSE**
ANALYSIS SYSTEM FOR ANALYZING WATER AND WASTEWATER
SYSTÈME POUR L'ANALYSE D'EAU ET D'EAUX USÉES

(30) Priorität: 08.12.2014 DE 102014118138
(43) Veröffentlichungstag der Anmeldung: 18.10.2017
(60) Teilanmeldung: 17194930.8
(73) Patentinhaber: LAR Process Analysers AG, 12057 Berlin (DE)
(72) Erfinder: ARTS, Werner, 10825 Berlin (DE); GENTHE, Wolfgang, 14059 Berlin (DE)
(74) Vertreter: Heinze, Ekkehard
(86) Internationale Anmeldenummer: PCT/DE2015/100519
(87) Internationale Veröffentlichungsnummer: WO 2016/091252

(56) Entgegenhaltungen:
- EP-A2- 1 022 564
- WO-A1-2006/019297
- WO-A2-93/17333
- US-A- 4 228 922

## Beschreibung

Die Erfindung betrifft eine Analyseanordnung zur Wasser- und Abwasseranalyse, die ein Analysegerät mit einem Gerätegehäuse und einem Injektionsport zur Einführung einer Probe in das Gerät sowie eine Injektionsspritze umfasst.

Derartige Analyseanordnungen sind an sich bekannt, z.B. aus der EP1022564A2, und werden auch von der Anmelderin hergestellt und angeboten.

Der Erfindung liegt die Aufgabe zu Grunde, eine verbesserte Analyseanordnung dieser Art bereitzustellen, die insbesondere verlässlich genaue und exakt reproduzierbare Analyseergebnisse liefern kann.

Diese Aufgabe wird nach einem ersten Aspekt der Erfindung gelöst durch eine Analyseanordnung mit den Merkmalen des Anspruchs 1.

Die Erfindung schließt zunächst den Gedanken ein, die zugehörige Injektionsspritze, abweichend von herkömmlichen Injektionsspritzen, im Interesse einer optimalen und reproduzierbaren Durchführung des Injektionsvorganges zu modifizieren. Dabei ist eine Austrittsöffnung der Nadel vorgesehen, deren Flächennormale mit der Nadel-Längsachse zusammenfällt. Weiter ist ein selbsttätig wirkendes Ausstoßelement zum Ausstoßen einer vorbestimmten Stoffmenge in einer vorbestimmten Injektionszeitspanne vorgesehen.

In der Erfindung ist der Injektionsport mit einem zylindrischen Reaktionsgefäß zum thermischen Aufschluss des zu analysierenden Stoffes verbunden, und er weist Führungsmittel zum Führen der Injektionsspritze in eine vorbestimmte Einspritzposition auf. Speziell sind die Führungsmittel in Abstimmung auf die Form der Injektionsnadel derart ausgebildet, dass die Längsachse der eingeführten Injektionsnadel mit der Längsachse des Reaktionsgefäßes zusammenfällt. Die Führungsmittel umfassen eine zylindrische oder konische Führungshülse. Weiter weisen die Führungsmittel einen Anschlag zur Begrenzung der Tiefe des Eindringens der Injektionsnadel in das Reaktionsgefäß auf.

In einer weiteren Ausführung der Erfindung ist das Ausstoßelement als verriegelbare Druckfeder ausgebildet, die auf den Kolben der Injektionsspritze wirkt. Die Druckfeder, die beispielsweise als stählerne Zylinderfeder ausgebildet sein kann, ersetzt also eine manuelle Betätigung der Spritze. Anders als bei der manuellen Betätigung, sichert die vorbestimmte Federcharakteristik der Druckfeder einen exakt reproduzierbaren Probenausstoß pro Zeiteinheit. Die Realisierung dieser wichtigen Wirkung muss aber nicht notwendigerweise durch eine Druckfeder (als besonders einfache und kostengünstige Ausführung) erfolgen, sondern kann beispielsweise auch durch einen kleinen Linearmotor oder einen hydraulischen oder pneumatischen Antrieb mit vorbestimmter Ausstoßcharakteristik erfolgen.

Vorteile und Zweckmäßigkeiten der Erfindung ergeben sich im Übrigen aus der nachfolgenden kurzen Beschreibung eines Ausführungsbeispiels anhand der Figuren. Von diesen zeigen:
Fig. 1 eine schematische Darstellung wesentlicher Gerätekomponenten eines Analysegerätes gemäß einem Ausführungsbeispiel,
Fig. 2 eine perspektivische Darstellung jenes Analysegerätes,
Fig. 3 eine schematische Längsschnittdarstellung einer Injektionsspritze mit in den Injektionsport eingeführter Injektionsnadel gemäß einer beispielhaften Ausführungsform der Analyseanordnung und
Fig. 4a und 4b grafische Darstellungen zur Illustration eines Aspekts der Erfindung.

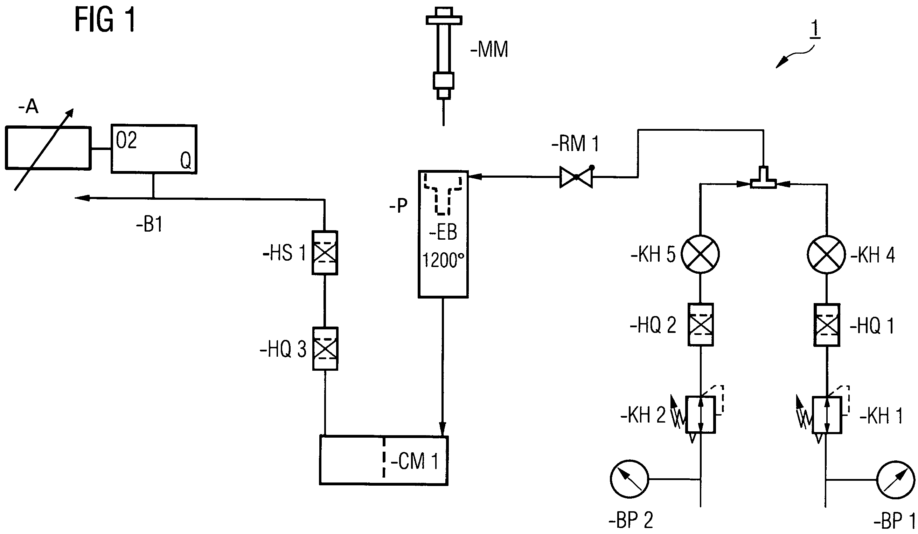

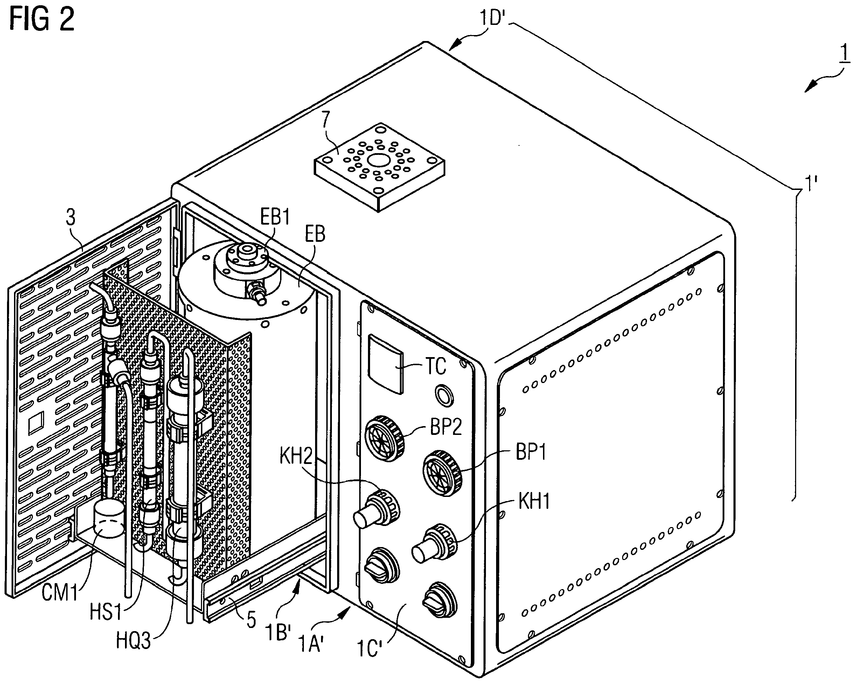

Fig. 1 und 2 zeigen schematisch wesentliche Teile eines Analysegerätes 1 zur Bestimmung des chemischen Sauerstoffbedarfs (CSB oder COD = chemical oxygen demand) von Wasser oder Abwasser. Der grundsätzliche Aufbau derartiger Geräte und deren Funktionsweise sind dem Fachmann bekannt und werden daher hier nicht näher beschrieben, zumal es hierauf zum Verständnis der vorliegenden Erfindung nicht ankommt.

Das Analysegerät 1 umfasst ein thermisches Reaktionsgefäß bzw. einen Ofen EB, in den mittels einer Injektionsspritze MM über einen an der Ofen-Oberseite angeordneten Injektionsport P eine Wasserprobe eingespritzt werden kann und in dem diese Probe thermisch aufgeschlossen wird. Dem Ofen wird über ein Rückschlagventil RM1 ein Trägergasstrom zugeführt, welcher aus Luft und Stickstoff zusammengesetzt wird. Der Trägergasstrom wird mittels eines Luft-Druckreglers KH1, eines Stickstoff-Druckreglers KH2, eines Luft-Durchflussreglers KH4 und eines Stickstoff-Durchflussreglers KH5 gesteuert und mittels eines ersten und zweiten Feinfilters HQ1, HQ2 eingangsseitig des Ofens gefiltert. Eingangsseitig des Ofens sind auch eine Luft-Druckanzeige BP1 und eine Stickstoff-Druckanzeige BP2 vorgesehen.

Ausgangsseitig des Ofens gelangt der Gasstrom zunächst in ein Kondensatgefäß CM1, und der nicht kondensierte Anteil durchläuft anschließend ein Quarzwollefilter HQ3 und eine Säurefalle HS1, bevor er zum Sauerstoffdetektor B1 gelangt, welcher schließlich einen (elektrischen) Messwert an eine einstellbare Auswertungseinrichtung A ausgibt, bei der insbesondere eine Integrationszeit zur Aufintegration eines zeitabhängig erfassten Sauerstoff-Nachweissignals vorgesehen ist; siehe dazu weiter unten.

Fig. 2 zeigt das Analysegerät 1 in perspektivischer Darstellung in einem Zustand, in dem das Gerätegehäuse 1' teilweise geöffnet ist und ein Teil der Gerätekomponenten aus dem Gehäuse herausgezogen ist. Das Gerätegehäuse hat im Wesentlichen die Gestalt eines quadratischen Prismas, und in der Gerätefront 1A' ist eine Öffnung 1B' vorgesehen, die durch eine an der linken Seitenkante der Gerätefront angeschlagene, perforierte Tür 3 zu verschließen ist.

Ein in seinen Abmessungen an die Öffnung 1B' angepasster Schlitten 5, auf dem der Ofen EB, das Kondensatgefäß CM1, das Quarzwollefilter HQ3 und die Säurefalle HS1 angeordnet sind, ist so weit aus dem Gehäuse herausziehbar, dass die genannten Komponenten frei zugänglich werden. Im zurückgeschobenen Zustand des Schlittens 5 wird das Gerätegehäuse 1' mit der Tür 3 verschlossen.

Auf der rechten Seite der Gerätefront 1A' befindet sich ein Bedienfeld 1C', auf dem mehrere Bedien- und Anzeigeelemente angeordnet sind, darunter eine Temperaturanzeige /-steuerung TC und die Einstellregler KH1 und KH2 für den Luft- bzw. Stickstoffdruck und die zugehörigen Anzeigeelemente BP1 und BP1.

Auf der Geräteoberseite 1D' befindet sich der Injektionsport P, dessen Aufbau und Abmessungen an diejenigen der in Fig. 1 gezeigten Injektionsspritze MM angepasst sind und der im Geräteinneren mit einem entsprechenden Einspritzventil EB1 des Ofens EB kommuniziert, wenn der Ofen sich in seiner normalen Gebrauchslage innerhalb des Gerätegehäuses befindet.

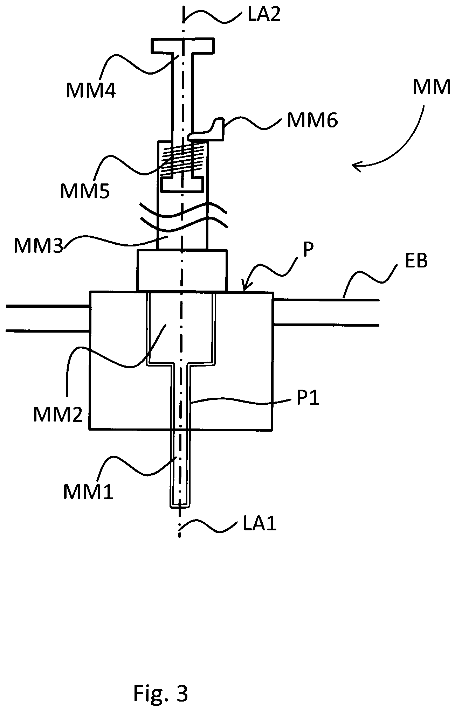

Fig. 3 zeigt skizzenartig in einer Längsschnittdarstellung den grundsätzlichen Aufbau und die aufeinander abgestimmte geometrische Konfiguration der Injektionsspritze MM und des Injektionsports P des Ofens EB der Analyseanordnung. Der Injektionsport P umfasst eine in ihrem Längsverlauf im Wesentlichen zylindrisch ausgebildete Führungshülse P1, deren Durchmesser und Länge auf die entsprechenden Abmessungen einer Injektionsnadel MM1 der Injektionsspritze MM abgestimmt sind und deren Längsachse mit einer Längsachse LA1 des in seiner Grundform zylindrisch ausgeführten Ofens EB zusammenfällt. An der Oberseite des Injektionsports P ist eine Bohrung P2 mit vergrößertem Durchmesser vorgesehen, deren Abmessungen auf diejenigen eines Nadel-Ansatzes MM2 der Injektionsspritze angepasst sind und deren untere Stirnfläche beim Einführen der Injektionsspritze als Anschlag zur Tiefenbegrenzung wirkt. Mit diesem Anschlag wird eine exakt vorbestimmte Position des rechtwinklig zur Nadel-Längsachse LA2 der Injektionsspritze abgeschnittenen Nadelendes im Ofen EB und somit ein exakt vorbestimmter Einspritzpunkt gewährleistet.

Im Spritzen-Reservoir MM3 ist ein Spritzenkolben MM4 längsverschieblich gelagert, dessen freies Ende in üblicher Weise zum manuellen Aufziehen einer Probe ausgestaltet ist. Am oberen Ende des Spritzenreservoirs ist in diesem eine Druckfeder MM5 eingebettet, deren oberes Ende sich gegen die obere Stirnwand des Spritzenreservoirs abstützt und deren unteres Ende auf das Ende des Spritzenkolbens MM4 wirkt. Nach dem Aufziehen der Spritze wird mittels eines Verriegelungshebels MM6 der Spritzenkolben mit gespannter Feder MM5 arretiert. Nach Lösen der Verriegelung MM6 wird der Spritzenkolben MM4 durch die Kraft der Druckfeder MM5 nach unten gedrückt und die im Spritzenreservoir MM3 enthaltene Probe in einem vorbestimmten Zeitintervall bzw. mit vorbestimmter Austraggeschwindigkeit in den Ofen eingespritzt.

Dieser Austrag der vorbestimmten Probenmenge mit exakt vorbestimmter Geschwindigkeit bzw. in einem exakt definierten Zeitintervall ist für reproduzierbare Analyseergebnisse ebenso wichtig wie die exakte Einspritzposition- und Richtung, die durch die spezielle Gestaltung der Injektionsnadel und des Injektionsports gesichert werden.

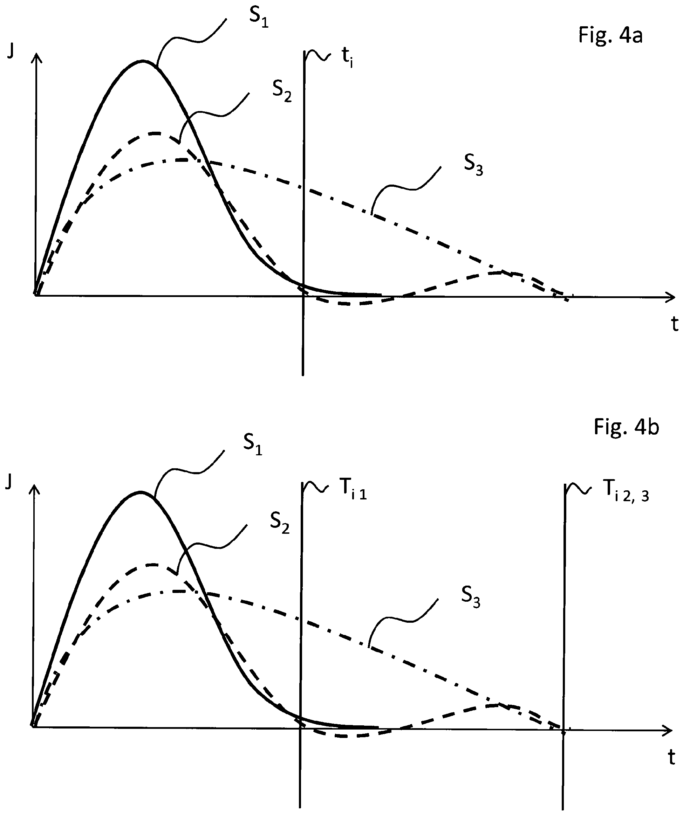

Fig. 4a und 4b illustrieren die Wirkung einer einstellbaren Integrationszeit der Nachbearbeitungseinrichtung A zur Verarbeitung eines zeitabhängig aufgenommenen Sauerstoff-Messsignals. Fig. 4a zeigt hierbei drei verschiedene Kurvenformen mit (gemäß dem Stand der Technik) fest eingestellter Integrationszeit tᵢ. Es ist erkennbar, dass hier nur die Aufintegration des mit der durchgezogenen Linie dargestellten Messsignals S₁ zu einem korrekten Ergebnis führt, während die eingestellte Integrationszeit für die Messsignale S₂ und S₃ offensichtlich zu kurz ist und wesentliche Signalanteile nicht erfasst werden. Abhilfe schafft hier das in Fig. 4b gezeigte Einstellen einer längeren Integrationszeit t_{i2, 3} für die Signale S₂ und S₃, die der Bediener der Analyseanordnung in Abhängigkeit der festgestellten Signalkurvenform an der Nachbearbeitungseinrichtung A vornehmen kann.

Die Ausführung der Erfindung ist nicht auf dieses Beispiel beschränkt, sondern ebenso in einer Vielzahl von Abwandlungen möglich, die im Rahmen des Anspruchs liegen.

## Patentansprüche

1. Analyseanordnung zur Wasser- und Abwasseranalyse, umfassend ein Analysegerät mit
einem Gerätegehäuse, welches Gerätekomponenten aufnimmt und welches an einer Gehäusefläche eine als Injektionsport ausgebildete Eintragöffnung zum Eintragen eines zu analysierenden Stoffes in eine Gerätekomponente bei geschlossenem Gerätegehäuse hat, und
einer Injektionsspritze, welche eine Austrittsöffnung der Injektionsnadel hat, deren Flächennormale mit der Längsachse zusammenfällt, und welche ein selbsttätig wirkendes Ausstoßelement zum Ausstoßen einer vorbestimmten Stoffmenge in einer vorbestimmten Injektionszeitspanne aufweist,
wobei der Injektionsport mit einem zylindrischen Reaktionsgefäß zum thermischen Aufschluss des zu analysierenden Stoffes verbunden ist und Führungsmittel zum Führen der Injektionsspritze in eine vorbestimmte Einspritzposition aufweist, wobei die Führungsmittel in Abstimmung auf die Form der Injektionsnadel derart ausgebildet sind, dass die Längsachse der eingeführten Injektionsnadel mit der Längsachse des Reaktionsgefäßes zusammenfällt, und eine zylindrische oder konische Führungshülse und einen Anschlag zur Begrenzung der Tiefe des Eindringens der Injektionsnadel in das Reaktionsgefäß aufweisen, wobei an der Oberseite des Injektionsports eine Bohrung mit vergrößertem Durchmesser vorgesehen ist, deren Abmessungen auf diejenigen eines Nadel-Ansatzes der Injektionsspritze angepasst sind und deren untere Stirnfläche als Anschlag zur Begrenzung der Tiefe des Eindringens der Injektionsnadel in das Reaktionsgefäß wirkt.

## Claims

1. Analysis system for analyzing water and waste water, comprising an analysis device with
an apparatus housing which accommodates apparatus components and which has on a housing surface an injection opening designed as an injection port for introducing a substance to be analysed into an apparatus component when the apparatus housing is closed, and
an injection syringe which has an outlet opening of the injection needle whose surface normal coincides with the longitudinal axis, and which has an automatically acting ejection element for ejecting a predetermined quantity of substance in a predetermined injection time period,
wherein the injection port is connected to a cylindrical reaction vessel for thermal digestion of the substance to be analyzed and comprises guide means for guiding the injection syringe into a predetermined injection position, the guide means being formed in accordance with the shape of the injection needle in such a way that the injection needle is able to be inserted into the injection port, such that the longitudinal axis of the inserted injection needle coincides with the longitudinal axis of the reaction vessel, and comprise a cylindrical or conical guide sleeve and a stop for limiting the depth of penetration of the injection needle into the reaction vessel, wherein an enlarged diameter bore is provided on the upper side of the injection port, whose dimensions are adapted to those of a needle hub of the injection syringe and whose lower end face acts as a stop for limiting the depth of penetration of the injection needle into the reaction vessel.

## Revendications

1. Système d'analyse destiné à l'analyse d'eau et d'eau usée, incluant un appareil d'analyse avec
un boîtier d'appareil, qui loge des composants d'appareil et qui présente sur une surface de boîtier une ouverture d'introduction, réalisée sous forme d'orifice d'introduction, pour l'entrée d'un produit à analyser jusque dans un composant de l'appareil alors que le boîtier est fermé, et
une seringue d'injection, qui possède une ouverture de sortie de l'aiguille d'injection, dont la normale à la surface coïncide avec l'axe longitudinal, et qui comprend un élément d'expulsion à action autonome pour l'éjection d'une quantité de produit prédéterminée dans une période temporelle d'injection prédéterminée,
dans lequel l'orifice d'injection est relié à un récipient de réaction cylindrique pour la dissociation thermique du produit à analyser et comprend un moyen de guidage pour guider la seringue d'injection jusque dans une position d'injection prédéterminée, dans lequel le moyen de guidage est réalisé en accord avec la forme de l'aiguille d'injection de telle façon que l'axe longitudinal de l'aiguille d'injection introduite coïncide avec l'axe longitudinal du récipient de réaction, et comprend une douille de guidage cylindrique ou conique ainsi qu'une butée pour limiter la profondeur de pénétration de l'aiguille d'injection dans le récipient de réaction, dans lequel il est prévu au niveau de la surface de l'orifice d'injection un perçage avec un diamètre agrandi, dont les dimensions sont ajustées à celle d'une souche de l'aiguille de la seringue d'injection, et dont la surface frontale inférieure fait office de butée pour limiter la profondeur de pénétration de l'aiguille d'injection dans le récipient de réaction.
